# EUROPEAN PATENT APPLICATION

(11) **EP 4 841 583 A1**
(43) Date of publication of application: **18.11.2026**
(21) Application number: 25176658.0
(22) Date of filing: 15.05.2025
(51) Int. Cl.: G06V 10/82, G06V 40/18, G06V 40/10

(54) **ESTIMATING BODY PART SIZE**

(71) Applicant: Koninklijke Philips N.V., 5656 AE Eindhoven (NL)
(72) Inventor: KULKAMI, Nikhil Vikram, Eindhoven (NL); BUSSA, Nagaraju, Eindhoven (NL); MEDEMA, Janine Willemijn, Eindhoven (NL)
(74) Representative: Philips Intellectual Property & Standards

(57) **Abstract**

Proposed concepts thus aim to provide schemes, solutions, concept, designs, methods and systems pertaining to estimating an absolute size of a body part. In particular, embodiments aim to provide a method for estimating an absolute size of a body part by using a neural network to detect an iris of the subject in the image, the size of which is standardized across the typical human and thus can essentially be used as an absolute ruler for estimating the absolute size of other body parts.

## Description

### FIELD OF THE INVENTION

This invention relates to the field of estimating an absolute size of a body part.

### BACKGROUND OF THE INVENTION

Accurately measuring body parts from a single image would have many practical uses in varied domains such as personal health, medical health, and e-commerce. It is known that the length of body parts in an image can be measured using a known focal length of the camera used to capture the image or a known height of the subject, but this requires prior knowledge to work. Furthermore, it is also known to use the ratios of body parts and categorize them into buckets instead of accurately measuring them, but this results in inaccurate and unreliable estimates.

### SUMMARY OF THE INVENTION

The invention is defined by the claims.

According to examples in accordance with a first aspect of the invention, there is provided a computer-implemented method for estimating an absolute size of a body part of a subject.

The method comprises: receiving image data representative of an image depicting a face and the body part of the subject; processing the image data with a neural network to detect an iris of the subject; determining a first size of the detected iris as represented in the image; detecting the body part in the image; determining a first size of the detected body part as represented in the image; and estimating the absolute size of the detected body part based on the first size of the detected body part, the first size of the detected iris and a predetermined absolute iris size.

Proposed concepts thus aim to provide schemes, solutions, concepts, designs, methods and systems pertaining to estimating an absolute size of a body part. In particular, embodiments aim to provide a method for estimating an absolute size of a body part by using a neural network to detect an iris of the subject in the image, the size of which is standardized across the typical human and thus can essentially be used as an absolute ruler for estimating the absolute size of other body parts.

The inventors have realized that as the width of an iris is standardized across the majority of humans, regardless of sex or age, it can essentially be used as a ruler against which to measure the size of other body parts present in the image. This works without having to know any prior knowledge specific to the subject or the image, such as focal length or subject height, because it can be assumed that all body parts will be substantially at the same depth in the image as the iris and thus their relative sizes in the image will be comparable.

Furthermore, the inventors have found that the use of a neural network allows an iris of a subject to be detected (and measured) from a single image in an effective and efficient manner, therefore facilitating the quick and automatic estimation of the absolute size of a body part present in the image.

In summary, the present method allows for an accurate estimation of the size of body parts without requiring specific prior knowledge such as focal length or subject height or specialized equipment such as a depth perception camera.

Ultimately, an improved method for estimating an absolute size of a body part is provided.

In an embodiment, processing the image data with the neural network to detect the iris of the subject comprises using one or more neural networks configured to: detect the face of the subject in the image; determine contours of an eye of the subject within the detected face; and determine contours of an iris of the subject within the determined contours of the eye; and wherein determining the first size of the detected iris may be based on the determined contours of the iris. This provides a particularly effective structure for first detecting and then determining the size of an iris using a neural network to ensure reliable detection and size determination.

In an embodiment, detecting the face of the subject comprises determining a bounding box for the face using a single shot multibox detector. A single shot multibox detector has been found to be especially effective for detecting the face of a subject in an image.

In an embodiment, detecting the body part in the image comprises: generating a body skeleton for the subject, the body skeleton comprising a plurality of landmarks and connecting lines between the landmarks, based on the image data. The connecting lines between the landmarks represent a first size of a corresponding body part. Detecting the body part in the image comprises detecting the body part based on the generated body skeleton. Determining the first size of the detected body part is based on the length of a connecting line corresponding to the body part.

In an embodiment, a mask RCNN model is used to generate the body skeleton. A mask RCNN model has been found to be especially effective for generating a body skeleton of a subject based on a single image.

In an embodiment, a body part comprises at least one of: an upper arm; a lower arm; a torso; shoulders; hips; an upper leg; a lower leg; and an entire body. These are all particularly useful body parts to determine the absolute size of, especially for personal care uses.

In an embodiment, the method further comprises: detecting an interpupillary distance of the subject in the image; determining a first size of the detected interpupillary distance as represented in the image; and wherein estimating the absolute size of the detected body part is further based on the determined first size of the detected interpupillary distance and an absolute size of the interpupillary distance. Additionally using the interpupillary distance essentially allows the method to be calibrated and thus facilitates a more reliable estimation of the absolute size of the body part.

In an embodiment, the method further comprises retrieving the absolute size of the interpupillary distance from a database based on the subject's age and sex. The size of an interpupillary distance is typically a standard value for a given sex and age, and thus its absolute size may be assumed for the present purpose.

In an embodiment, the method further comprises estimating the absolute size of the interpupillary distance based on the first size of the detected interpupillary distance, the first size of the detected iris and the predetermined iris size. This facilitates obtaining the absolute size of the interpupillary distance without having to know the age and sex of the subject.

In an embodiment, the method further comprises determining an operating parameter of a personal care device based on the estimated absolute size of the body part. This facilitates the at least partial automation of a personal care device for which body part size may be an especially useful parameter to know.

In an embodiment, the personal care device may comprise an Intense Pulsed Light, IPL, device, and the operating parameter may comprise a number of flashes to be used on the body part. The absolute size of a body part on which the IPL device is to be used may be especially useful knowledge for this specific purpose.

In an embodiment, the image of the subject may be a two-dimensional image. It is particularly useful that that the image only needs to be two-dimensional therefore allowing the image to be captured with a typical camera, e.g., a smartphone camera.

According to a second aspect of the invention, there is provided a computer program comprising code means which, when executed on a processor, cause the computer-implemented method of the first aspect to be performed.

According to a third aspect of the invention, there is provided a system for estimating an absolute size of a body part. The system comprises a processor configured to execute the computer program product according to the second aspect.

For example, the system is a mobile device operated by the user. In another example, the system is a server adapted to perform computation in the cloud.

According to a fourth aspect of the invention, there is provided a light-based skin treatment device adapted to perform skin treatment. The light-based skin treatment device comprises a light source, a controller, and a data input. The light source is adapted to generate treatment light to perform skin treatment. The data input is adapted to receive input data from the system according to the third aspect. The data input is representative of the estimated absolute size of the detected body part. The controller is adapted to control the light-based skin treatment device to perform skin treatment based on the estimated absolute size of the detected body part.

For example, the light-based skin treatment device is an Intense Pulsed Light, IPL, device. For example, the IPL device is adapted to perform photo depilation.

The light source is adapted to generate treatment light. For example, the light source is adapted to provide light pulses, also referred to as flashes. For example, the light source is adapted to generate light at a high intensity for a short duration, such as for less than 100 ms or less than 50 ms or less than 10 ms or less than 8 ms. The intensity of the light pulse is high enough to perform treatment on the skin. Such treatment is, for example, hair removal, or photo-rejuvenation, or vein treatment, or acne treatment. For example, the light source comprises one or more optical filters. For example, the light source comprises an optical filter to prevent light with a potentially damaging wavelength, such as UV light, from being transmitted to the skin. For example, the light source comprises a Xenon flash lamp, or a laser, or multiple lasers, a LED, or multiple LEDs. For example, the light source is adapted to provide treatment light with wavelengths in the range of 530-1200 nanometers.

The data input is adapted to receive the input data via a wire or wireless. For example, the data input is adapted to receive the input data via Bluetooth, or WiFi, or any other type of wireless communication.

The controller is adapted to control the light-based skin treatment device based on the estimated absolute size of the detected body part. For example, the controller controls a number of flashes of treatment light generated by the light source. For example, the controller controls the intensity of the treatment light. For example, the controller controls a user guidance to guide the user to move the light-based skin treatment device.

Thus, there are proposed concepts for estimating an absolute size of a body part, and this is done based on using a neural network to detect an iris of a subject in an image, an absolute size of which is standardized and thus can be used to help estimate the absolute size of another body part in the image.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 is a simplified flow diagram of a method for estimating an absolute size of a body part according to a first embodiment;
Fig. 2 is a flow diagram of a method for estimating an absolute size of a body part according to a second embodiment;
Fig. 3 is a flow diagram of a method for estimating an absolute size of a body part including generating a body skeleton according to a third embodiment;
Fig. 4 is a flow diagram of a method for estimating an absolute size of a body part including using an interpupillary distance according to a fourth embodiment;
Fig. 5 is a simplified block diagram of a system for estimating an absolute size of a body part according to a fifth embodiment; and
Fig. 6 illustrates an example of a computer within which one or more parts of an embodiment may be employed.
Fig. 7 depicts a light-based skin treatment device according to a sixth embodiment;
Fig. 8 depicts a detail of the light-based skin treatment device according to the sixth embodiment.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

Implementations in accordance with the present disclosure relate to various techniques, methods, schemes and/or solutions pertaining to estimating an absolute size of a body part. According to proposed concepts, a number of possible solutions may be implemented separately or jointly. That is, although these possible solutions may be described below separately, two or more of these possible solutions may be implemented in one combination or another.

Embodiments of the invention aim to provide a method for estimating an absolute size of a body part. This can be achieved by using a neural network to detect an iris of the subject in the image, the size of which is standardized across the typical human and thus can essentially be used as an absolute ruler for estimating the absolute size of other body parts.

The inventors have realized that as the width of an iris is a standardized across typical humans, regardless of sex or age, it can essentially be used as a ruler against which to measure the size of other body parts present in the image. This works without having to know any prior knowledge specific to the subject/image, such as focal length or subject height, because it can be assumed that all body parts will be substantially at the same depth in the image as the iris and thus their relative sizes in the image will be comparable.

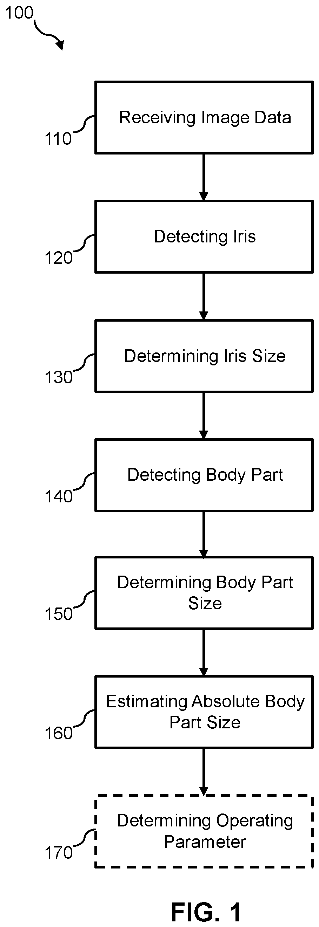

Referring now to Fig. 1, there is depicted a simplified flow diagram of a computer-implemented method 100 for estimating an absolute size of a body part according to a first embodiment.

The method 100 begins with the step 110 of receiving image data representative of an image of a subject including at least their face and the body part (i.e., the body part of which the absolute size is to be estimated). The image including their face is to be understood as the image including at least a portion of their face which includes an eye, however, preferably the image includes their full face. It should also be noted that the image should be a single intact image, i.e., not a composite image, and should be taken using a consistent focal length across the entire image.

In this embodiment, the body part comprises at least one of: an upper arm; a lower arm; a torso; shoulders; hips; an upper leg; a lower leg; and an entire body. These are all particularly useful body parts to determine the absolute size of, especially for personal care uses such as depilation, though of course, as the skilled person would appreciate, in other embodiments, the body part can comprise any body part of sufficient size (i.e., of a size equal to or larger than an iris).

Furthermore, in this embodiment, the image of the subject is a two-dimensional image (as opposed to a 3D or depth image). It is particularly useful that that the image only needs to be two-dimensional therefore allowing the image to be captured with a typical camera, e.g., a smartphone camera.

Step 120 comprises processing the image data with a neural network to detect an iris of the subject (i.e., on the subject's face which is present in the image). To be suitable for this purpose, the neural network must be a neural network which is configured (i.e., has been trained) to detect irises in images. This step can thus otherwise be understood as detecting an iris of the subject in the image using a neural network.

Step 130 comprises determining a first size of the detected iris as represented in the image. That is, the first size is a relative size (not an absolute size) which describes the size of the detected iris in terms relative to the image (e.g., using pixels or a percentage of the image length/width, etc.). In this embodiment, the size of the iris being determined is specifically the horizontal width of the iris however, in other embodiments, the size may be the vertical height of the iris or the area of the iris. Step 130 can be performed by the same neural network as used in step 120 or a different neural network or a machine-learning algorithm or a non-machine-learning algorithm - the skilled person would be aware that there are many ways in which the first size could be determined.

Step 140 comprises detecting the body part (to-be-sized) in the image. The skilled person would be aware of the myriad ways in which this could be achieved, for example, by applying machine learning-based image recognition, edge detection algorithms, key-point estimation techniques, etc.

Step 150 comprises determining a first size (i.e., a first length, width, and/or area) of the detected body part as represented in the image. In the same way as for the first size of the detected iris, the first size of the detected body part is a relative size (not an absolute size) which describes the size of the detected body part in terms relative to the image (e.g., using pixels or a percentage of the image length/width, etc.).

Step 160 comprises estimating the absolute size (i.e., its real size (length, width, and/or area) in absolute units such as cm, inches, etc.) of the detected body part based on the first size of the detected body part, the first size of the detected iris and a predetermined absolute iris size. The diameter of an iris is typically constant (11.7±0.5 mm) across the vast majority of humans regardless of factors such as lighting, pupil dilation or changes in facial expression. While the pupil size may vary, the outer boundary of the iris does not change. This allows the iris to essentially act as an absolute scale in the image which can be used to calculate the absolute size of other body parts in the image which will be at roughly the same depth in the image as the iris, thus making their relative sizes comparable. Therefore, by using the determined relative sizes of the iris and the body part to-be-sized and the known size of the iris, the absolute size of the body part can thus be calculated.

In this embodiment, the method 100 comprises a further step 170 of determining an operating parameter of a personal care device based on the estimated absolute size of the body part. This facilitates the at least partial automation of a personal care device for which body part size may be an especially useful parameter to know. However, in other embodiments, step 170 need not be present (e.g., the absolute size of the body part can be estimated purely for knowledge purposes or it may be used in a different subsequent process).

In this embodiment, the personal care device specifically comprises an Intense Pulsed Light, IPL, device, and the operating parameter comprises a number of flashes to be used on the body part (of which the absolute size was determined). The absolute size of a body part may be especially useful knowledge for this specific purpose, though other purposes would be apparent to the skilled person. For instance, the personal care device may be a light-based hair removal device and the operating parameter may comprise the energy dosage or treatment duration for the body part. In another example, the personal care device may be an automatic massager and the operating parameter may comprise the duration to be applied to the body part.

In summary, in order to get the real-world dimension of an object using a 2D image, typically either specialized hardware incorporated into the camera itself is needed (such as a depth perception camera) or intrinsic/extrinsic properties of the camera need to be known such as focal length. Without any of these available, an object of known size in the image is needed to determine the size of a target object (which has to be at substantially the same depth as the object of known size). However, always having an object of known size in an image is challenging. In this invention, the fact that the horizontal iris diameter of the human eye remains roughly constant at 11.7±0.5 mm across a wide population is leveraged. The present invention is most suitable for any subject above 3 years of age, as the iris is known to stop growing at around 3 years of age. It has been found that using the present method, the length of body parts can be estimated within an accuracy of half a centimeter.

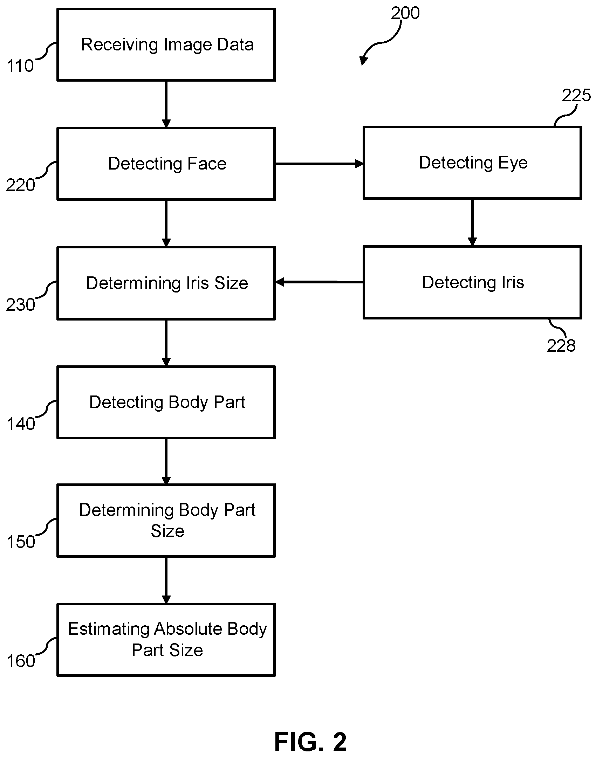

Referring now to Fig. 2, there is depicted a flow diagram of a computer-implemented method 200 for estimating an absolute size of a body part according to a second embodiment. Steps 110, 140, 150 and 160 are substantially the same the corresponding steps described in relation to method 100 of the first embodiment in Fig. 1.

In this method 200, compared to method 100, step 120 has essentially been replaced with steps 220, 225 and 228, which are performed using one or more neural networks specifically configured to carry the steps out. Step 220 comprises detecting the face of the subject in the image. The skilled person would be aware of the many ways this could be performed, for instance, using a Multi-task Cascaded Convolutional Network (MTCNN), employing Haar cascade classifiers, using Histogram of Oriented Gradients (HOG) with Support Vector Machines, or deep learning models like YOLO or SSD adapted for facial detection. In this embodiment, however, detecting the face of the subject specifically comprises determining a bounding box for the face using a single shot multibox detector. A single shot multibox detector has been found to be especially effective for detecting the face of a subject in an image.

Step 225 comprises determining contours of an eye of the subject within the detected face. In other words, step 225 comprises segmenting the eye region within the facial bounding box and extracting its geometric boundary or outline. The skilled person would be aware of the many ways this could be performed, for instance, by applying facial landmark detection models or regression-based models trained to output the coordinates of key eye landmarks, followed by, for example, spline fitting or convex hull estimation to derive contours.

Step 228 comprises determining contours of an iris of the subject within the determined contours of the eye. In other words, step 228 comprises segmenting the iris region within the eye by identifying its circular or elliptical boundary. The skilled person would be aware of the many ways this could be performed, for instance, by using a Circle Hough transform, integro-differential operators, deep learning-based semantic segmentation networks such as U-Net trained for iris boundary detection, or active contour models.

Step 230 comprises determining the first size of the detected iris based on the determined contours of the iris. For instance, the width of the contours of the iris can be taken as the relative (first) width of the iris.

In a specific example, of the invention, two hundred and thirty landmark features of the subject's face are detected after said face has been detected, e.g., in step 220. For example, in step 225, a landmark detector can be used which crops the image using the bounding box coordinates detected by a face detector in step 220, and then recognizes up to 230 landmarks on the subject's face, for example, nose, cheeks, mouth contours and of course eye contours using encoder-decoder architecture. Once the contours of the eyes have been located, the iris can be located within said contours. The landmark detector may save the coordinates (e.g., X-Y coordinates of the image) of its detected landmarks to a file for further analysis (and/or, for example, use by an iris detector). As the skilled person would be aware, the landmark detector can be trained on images of faces annotated with example landmarks.

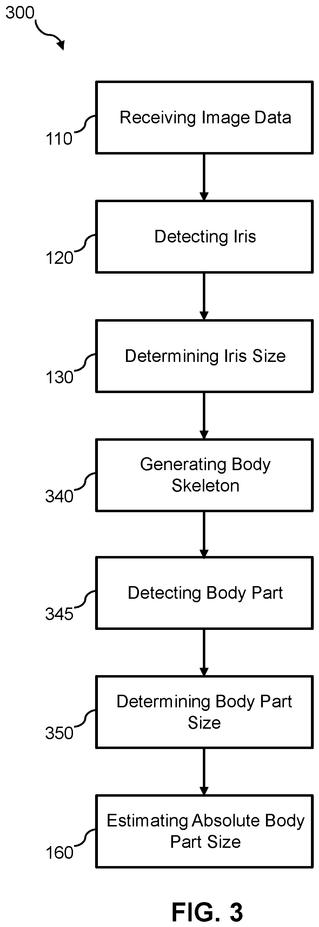

Referring now to Fig. 3, there is depicted a flow diagram of a computer-implemented method 300 for estimating an absolute size of a body part including generating a body skeleton according to a third embodiment. Steps 110, 120, 130 and 160 are substantially the same as have been described in relation to the method 100 of the first embodiment in Fig. 1.

Compared to method 100, in this method 300, step 140 has essentially been replaced with steps 340 and 345. Step 340 comprises generating a body skeleton for the subject, the body skeleton comprising a plurality of landmarks and connecting lines between the landmarks, based on the image. The connecting lines between the landmarks represent a first size (i.e., a relative size) of a corresponding body part. In other words, step 340 comprises constructing a model of the subject's pose in the image by identifying key anatomical points (such as joints) and determining the relative spatial distances using straight lines between these points to estimate the proportional dimensions of body segments. The skilled person would be aware of the many ways in which a body skeleton could be generated from an image of a subject. In this embodiment, however, a mask region-based convolutional neural network (RCNN) model is used to generate the body skeleton. As the skilled person would know, a Mask-RCNN outputs a mask of a target object, which in this case would be a person. In an example of the invention, a derivative of a mask-RCNN is used to facilitate landmark/keypoint detection. Landmark/keypoint RCNN slightly modifies an existing Mask RCNN by one-shot encoding a landmark/keypoint (instead of the whole mask) of the detected object. For instance, the modified model can be custom trained using annotated images and a Mask RCNN as backbone architecture. A model can then be developed which can detect all landmarks in an image in one shot. A mask RCNN model (or derivative thereof) has been found to be especially effective for generating a body skeleton of a subject based on a single image but other approaches can be used in other embodiments such as, for example, any key point detection framework based on convolutional neural networks or transformer architectures.

Step 345 comprises detecting the body part based on the generated body skeleton. For instance, if the body part were the subject's right upper arm, the body skeleton could be used to help localize the subject's right upper arm (e.g., the line between the right shoulder and the right elbow) and confirm its positioning and orientation. In other words, step 345 comprises identifying a specific body part (e.g., limb or region of the body) by referencing the landmarks and connecting lines of the body skeleton to isolate the spatial region corresponding to that body part.

Step 350 then comprises determining the first (relative) size of the detected body part based on the length of a connecting line corresponding to the body part. For instance, the relative size of the subject's right upper arm may be determined by determining the length of the line between the right shoulder landmark and the right elbow landmark in the body skeleton. In other words, step 350 comprises calculating the first (relative) size of the body part (to-be-sized) using the length of one or more lines between the appropriate landmarks in the body skeleton. The skilled person would appreciate how this could be done dependent on the landmarks involved in the body skeleton. For instance, to determine the length of a subject's left leg, the length of the line between the subject's left hip and left knee could be summed with the length of the line between the subject's left knee and left heel to arrive at a first length for the subject's left leg. It should be noted that the lengths of the lines of the body skeleton will be in terms relative to the image, for instance, pixel length or percentage of the image's length/width, etc.

In a specific example of the invention, a body skeleton is generated for the subject using keypoint detection. Keypoint detection entails estimating the position of unique points on the human body, such as joint points (e.g., elbows, knees, shoulders, etc.) or specific features such as eyes and/or ears. In this example, keypoint detection is performed on a single two-dimension image and the exact coordinates (e.g., X, Y coordinates of the image) of the keypoints are extracted. In this example, a total of 17 keypoints are detected: left and right ankle; left and right knee; left and right hip; left and right shoulder; left and right elbow; left and right wrist; left and right ear; left and right eye; and nose.

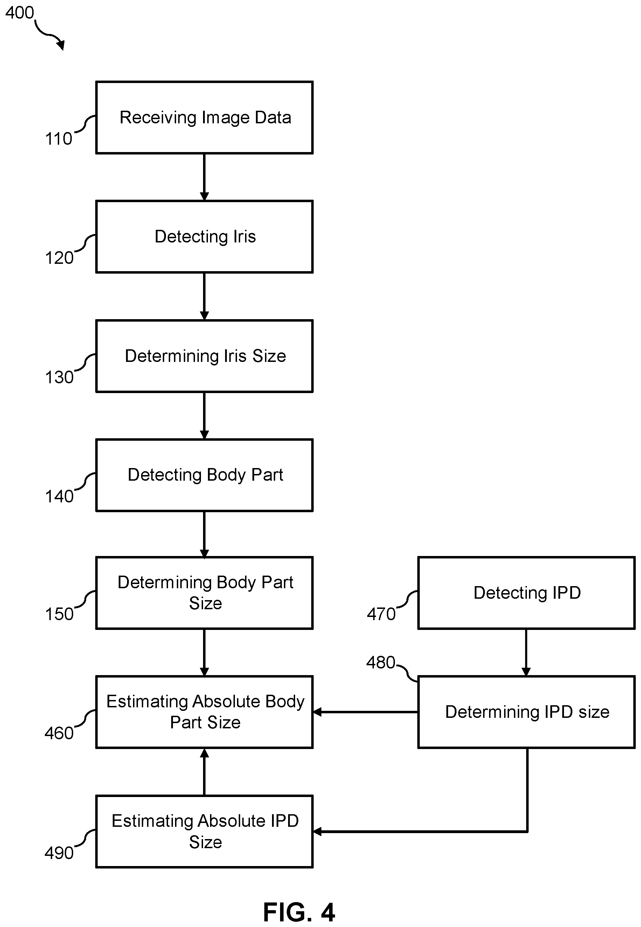

Referring now to Fig. 4, there is depicted a flow diagram of a computer-implemented method 400 for estimating an absolute size of a body part including using an interpupillary distance according to a fourth embodiment. Steps 110, 120, 130, 140 and 150 are substantially the same as have been described in relation to the method 100 of the first embodiment in Fig. 1.

Step 470 comprises detecting an interpupillary distance (IPD) of the subject in the image. As the skilled person would know, the IPD is the linear distance between the centers of the subject's pupils. The IPD of a subject can be detected in many ways, as the skilled person would be aware, such as by first detecting the eye regions using facial landmark detection, for example, and then determining a line between the coordinates of the centers of the pupils, which can be estimated via iris segmentation, reflection-based pupil detection, or through regression models trained to predict pupil locations directly from image data, etc. The first size of the detected IPD relative to the rest of the image can then be determined by calculating the length of the determined line, for example, relative to the rest of the image. For example, the length of the line can be determined in pixels or as a percentage of the image's length/width, etc.

In some embodiments, step 470 and 480 can be combined and performed in a single step such that the IPD is detected and its length/size determined simultaneously.

In step 460, the absolute size of the detected body part is estimated in substantially the same way as in step 160 of method 100, but with the estimation being further based on the determined first size of the detected interpupillary distance and an absolute size of the interpupillary distance. Additionally using the interpupillary distance essentially allows the method to be calibrated and thus facilitates a more reliable estimation of the absolute size of the body part. For example, the ratio between the first (relative) size of the IPD and its absolute size can be used to further inform what ratio should be present between the first (relative) size of the body part and its absolute size.

In some embodiments, the absolute size of the IPD can be retrieved from a database based on the subject's age and sex. The size of an interpupillary distance is typically a standard value for a given sex and age, and thus its absolute size can be assumed (given knowledge of the subject's age and sex) for the present purpose.

In this embodiment, however, the absolute size of the IPD is obtained via step 490 which comprises estimating the absolute size of the interpupillary distance based on the first size of the detected interpupillary distance, the first size of the detected iris and a predetermined iris size. This facilitates obtaining the absolute size of the interpupillary distance without having to know the age and sex of the subject.

In some embodiments, the absolute size of the IPD can be estimated in step 490 based on an additional image specifically of the subject's face such that the estimation of the absolute size of the IPD is made more accurate.

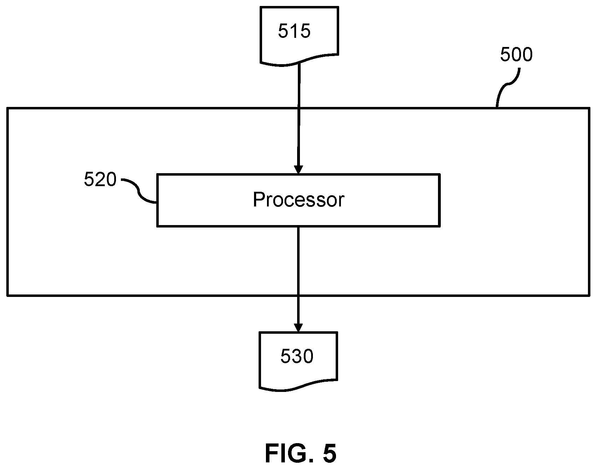

Referring now to Fig. 5, there is depicted a system 500 for estimating an absolute size of a body part according to a fifth embodiment. The system 500 comprises a processor 520.

The processor 520 can be configured to perform any herein-disclosed method (e.g., any of methods 100, 200, 300, or 400). However, in this embodiment specifically, the processor 520 is configured to perform method 100, i.e., to: receive image data representative of an image of a subject including at least their face and the body part; processing the image data with a neural network to detect an iris of the subject; determine a first size of the detected iris as represented in the image; detect the body part in the image; determine a first size of the detected body part as represented in the image; and estimate the absolute size of the detected body part based on the first size of the detected body part, the first size of the detected iris and the predetermined absolute iris size. The input 515 into the system 500 is thus image data of an image of the subject including at least their face and the body part. The output 530 of the system is an estimate of the absolute size of the detected body part. In some embodiments, the system 500 may further comprise an input interface configured to receive the image and pass it onto the processor 520.

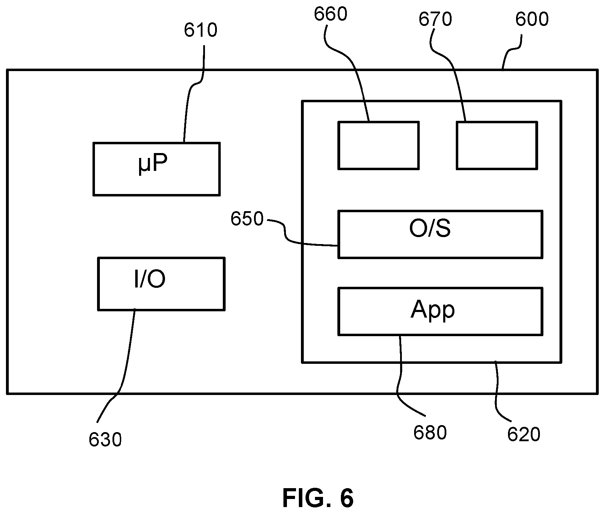

Fig. 6 illustrates an example of a computer 600 within which one or more parts of an embodiment may be employed. Various operations discussed above may utilize the capabilities of the computer 600. In this regard, it is to be understood that system functional blocks can run on a single computer or may be distributed over several computers and locations (e.g. connected via internet).

The computer 600 includes, but is not limited to, PCs, workstations, laptops, PDAs, palm devices, servers, storages, and the like. Generally, in terms of hardware architecture, the computer 600 may include one or more processors 610 (which include the processor 520), memory 620 and one or more I/O devices 630 that are communicatively coupled via a local interface (not shown). The local interface can be, for example but not limited to, one or more buses or other wired or wireless connections, as is known in the art. The local interface may have additional elements, such as controllers, buffers (caches), drivers, repeaters, and receivers, to enable communications. Further, the local interface may include address, control, and/or data connections to enable appropriate communications among the aforementioned components.

The processor 610 is a hardware device for executing software that can be stored in the memory 620. The processor 610 can be virtually any custom made or commercially available processor, a central processing unit (CPU), a digital signal processor (DSP), a graphical processing unit (GPU) or an auxiliary processor among several processors associated with the computer 600, and the processor 610 may be a semiconductor based microprocessor (in the form of a microchip) or a microprocessor.

The memory 620 can include any one or combination of volatile memory elements (e.g., random access memory (RAM), such as dynamic random access memory (DRAM), static random access memory (SRAM), etc.) and non-volatile memory elements (e.g., ROM, erasable programmable read only memory (EPROM), electronically erasable programmable read only memory (EEPROM), programmable read only memory (PROM), tape, compact disc read only memory (CD-ROM), disk, diskette, cartridge, cassette or the like, etc.). Moreover, the memory 620 may incorporate electronic, magnetic, optical, and/or other types of storage media. Note that the memory 620 can have a distributed architecture, where various components are situated remote from one another, but can be accessed by the processor 610.

The software in the memory 620 may include one or more separate programs, each of which comprises an ordered listing of executable instructions for implementing logical functions. The software in the memory 620 includes a suitable operating system (O/S) 650, compiler 660, source code 670, and one or more applications 680 in accordance with exemplary embodiments. As illustrated, the application 680 comprises numerous functional components for implementing the features and operations of the exemplary embodiments. The application 680 of the computer 600 may represent various applications, computational units, logic, functional units, processes, operations, virtual entities, and/or modules in accordance with exemplary embodiments, but the application 680 is not meant to be a limitation.

The operating system 650 controls the execution of other computer programs, and provides scheduling, input-output control, file and data management, memory management, and communication control and related services. It is contemplated by the inventors that the application 680 for implementing exemplary embodiments may be applicable on all commercially available operating systems.

Application 680 may be a source program, executable program (object code), script, or any other entity comprising a set of instructions to be performed. When a source program, then the program is usually translated via a compiler (such as the compiler 660), assembler, interpreter, or the like, which may or may not be included within the memory 620, so as to operate properly in connection with the O/S 650. Furthermore, the application 680 can be written as an object oriented programming language, which has classes of data and methods, or a procedure programming language, which has routines, subroutines, and/or functions, for example but not limited to, C, C++, C#, Pascal, Python, BASIC, API calls, HTML, XHTML, XML, ASP scripts, JavaScript, FORTRAN, COBOL, Perl, Java, ADA, .NET, and the like.

The I/O devices 630 may include input devices such as, for example but not limited to, a mouse, keyboard, scanner, microphone, camera, etc. Furthermore, the I/O devices 630 may also include output devices, for example but not limited to a printer, display, etc. Finally, the I/O devices 630 may further include devices that communicate both inputs and outputs, for instance but not limited to, a NIC or modulator/demodulator (for accessing remote devices, other files, devices, systems, or a network), a radio frequency (RF) or other transceiver, a telephonic interface, a bridge, a router, etc. The I/O devices 630 also include components for communicating over various networks, such as the Internet or intranet.

If the computer 600 is a PC, workstation, intelligent device or the like, the software in the memory 620 may further include a basic input output system (BIOS) (omitted for simplicity). The BIOS is a set of essential software routines that initialize and test hardware at start-up, start the O/S 650, and support the transfer of data among the hardware devices. The BIOS is stored in some type of read-only-memory, such as ROM, PROM, EPROM, EEPROM or the like, so that the BIOS can be executed when the computer 600 is activated.

When the computer 600 is in operation, the processor 610 is configured to execute software stored within the memory 620, to communicate data to and from the memory 620, and to generally control operations of the computer 600 pursuant to the software. The application 680 and the O/S 650 are read, in whole or in part, by the processor 610, perhaps buffered within the processor 610, and then executed.

When the application 680 is implemented in software it should be noted that the application 680 can be stored on virtually any computer readable medium for use by or in connection with any computer related system or method. In the context of this document, a computer readable medium may be an electronic, magnetic, optical, or other physical device or means that can contain or store a computer program for use by or in connection with a computer related system or method.

The application 680 can be embodied in any computer-readable medium for use by or in connection with an instruction execution system, apparatus, or device, such as a computer-based system, processor-containing system, or other system that can fetch the instructions from the instruction execution system, apparatus, or device and execute the instructions. In the context of this document, a "computer-readable medium" can be any means that can store, communicate, propagate, or transport the program for use by or in connection with the instruction execution system, apparatus, or device. The computer readable medium can be, for example but not limited to, an electronic, magnetic, optical, electromagnetic, infrared, or semiconductor system, apparatus, device, or propagation medium.

The methods of Figs. 1-4, and the system of Fig. 5, may be implemented in hardware or software, or a mixture of both (for example, as firmware running on a hardware device). To the extent that an embodiment is implemented partly or wholly in software, the functional steps illustrated in the process flowcharts may be performed by suitably programmed physical computing devices, such as one or more central processing units (CPUs) or graphics processing units (GPUs). Each process - and its individual component steps as illustrated in the flowcharts - may be performed by the same or different computing devices. According to embodiments, a computer-readable storage medium stores a computer program comprising computer program code configured to cause one or more physical computing devices to carry out an encoding or decoding method as described above when the program is run on the one or more physical computing devices.

Storage media may include volatile and non-volatile computer memory such as RAM, PROM, EPROM, and EEPROM, optical discs (like CD, DVD, BD), magnetic storage media (like hard discs and tapes). Various storage media may be fixed within a computing device or may be transportable, such that the one or more programs stored thereon can be loaded into a processor.

To the extent that an embodiment is implemented partly or wholly in hardware, the blocks shown in the block diagrams of Fig. 6 may be separate physical components, or logical subdivisions of single physical components, or may be all implemented in an integrated manner in one physical component. The functions of one block shown in the drawings may be divided between multiple components in an implementation, or the functions of multiple blocks shown in the drawings may be combined in single components in an implementation. Hardware components suitable for use in embodiments of the present invention include, but are not limited to, conventional microprocessors, application specific integrated circuits (ASICs), and field-programmable gate arrays (FPGAs). One or more blocks may be implemented as a combination of dedicated hardware to perform some functions and one or more programmed microprocessors and associated circuitry to perform other functions.

A single processor or other unit may fulfil the functions of several items recited in the claims. The mere fact that certain measures are recited in mutually different dependent claims does not indicate that a combination of these measures cannot be used to advantage. If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems. If the term "adapted to" is used in the claims or description, it is noted the term "adapted to" is intended to be equivalent to the term "configured to". Any reference signs in the claims should not be construed as limiting the scope.

The flowchart and block diagrams in the Figures illustrate the architecture, functionality, and operation of possible implementations of systems, methods, and computer program products according to various embodiments of the present invention. In this regard, each block in the flowchart or block diagrams may represent a module, segment, or portion of instructions, which comprises one or more executable instructions for implementing the specified logical function(s). In some alternative implementations, the functions noted in the block may occur out of the order noted in the figures. For example, two blocks shown in succession may, in fact, be executed substantially concurrently, or the blocks may sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts or carry out combinations of special purpose hardware and computer instructions, the architecture, functionality, and operation of possible implementations of systems, methods, and computer program products according to various embodiments of the present invention.

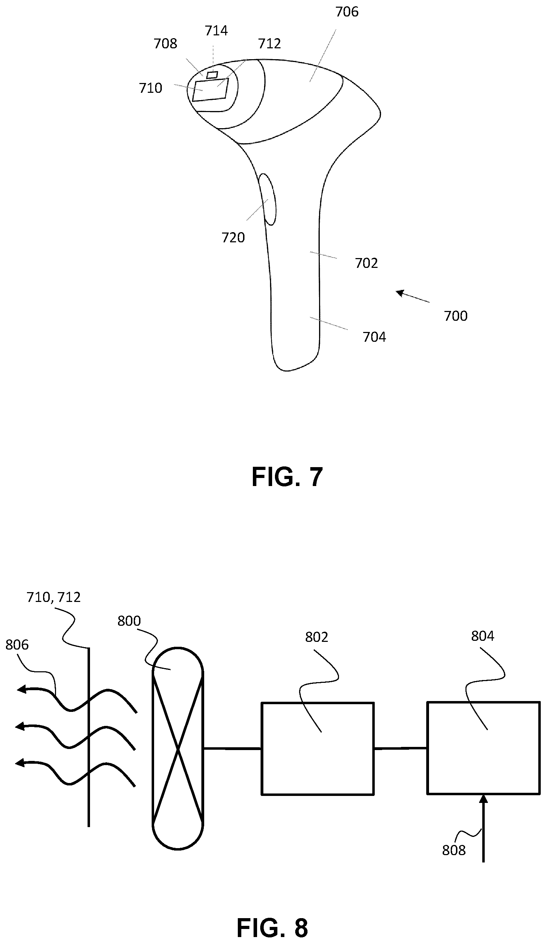

Fig. 7 depicts a light-based skin treatment device 700 according to a sixth embodiment. Fig. 8 depicts a detail of the sixth embodiment. The light-based skin treatment device 700 is adapted to perform skin treatment. The light-based skin treatment device 700 comprises a light source 800, a controller 802, and a data input 804. The light source 800 is adapted to generate treatment light 806 to perform skin treatment. The data input 804 is adapted to receive input data 808 from the system 500 according to the fifth embodiment. The input data 808 is representative of the estimated absolute size of the detected body part. The controller 802 is adapted to control the light-based skin treatment device 700 to perform skin treatment based on the estimated absolute size of the detected body part.

Fig. 7 depicts the light-based skin treatment device 700, in this case an Intense Pulsed Light, IPL, device. The light-based skin treatment device 700 is adapted for use on the skin of a subject, e.g. a person or an animal. The light-based skin treatment device 700 comprises a housing 702 that has a handle portion 704 and a head portion 706. The handle portion 704 is shaped to enable the user to hold the light-based skin treatment device 700. The head portion 706 has a head end 708 that is to be placed into contact with the subject in order for the light-based skin treatment device 700 to perform a treatment operation on the skin of the subject.

The light-based skin treatment device 700 comprises the light source 800 adapted to provide treatment light 806 to the skin. The light source 800 is arranged inside the head portion 706. The light-based skin treatment device 700 performs the treatment operation by providing pulses of the treatment light 806 to the skin via the aperture 710. The aperture 710 is arranged in or on the housing 702 so that the aperture 710 can be placed adjacent to or on (i.e. in contact with) the skin of the subject. An IPL window 712 is arranged in the aperture 710. The intensity of the light pulses from the treatment light source are high enough to perform the treatment operation on the skin or body part adjacent to the aperture 710.

The light-based skin treatment device 700 comprises a user control 720 adapted to be operated by the user to activate the IPL apparatus 700. When the user activates the IPL apparatus 100 via the user control 720, the IPL apparatus 700 is able to perform the treatment operation. The user control 720 may be in the form of a switch, a button, a touch pad, etc. Optionally, the light-based skin treatment device 700 has a sensor 714 to detect whether skin is present at the aperture 710.

## Claims

1. A computer-implemented method (100) for estimating an absolute size of a body part of a subject, the method comprising:
receiving image data (100) representative of an image depicting a face and the body part of the subject;
processing the image data (120) with a neural network to detect an iris of the subject;
determining a first size of the detected iris (130) as represented in the image;
detecting the body part (140) in the image;
determining a first size of the detected body part (150) as represented in the image; and
estimating the absolute size of the detected body part (160) based on the first size of the detected body part, the first size of the detected iris, and a predetermined absolute iris size.

2. The computer-implemented method of claim 1, wherein processing the image data with the neural network to detect the iris of the subject comprises using one or more neural networks configured to:
detect the face of the subject (220) in the image;
determine contours of an eye of the subject (225) within the detected face; and
determine contours of an iris of the subject (228) within the determined contours of the eye; and
wherein determining the first size of the detected iris is based on the determined contours of the iris (230).

3. The computer-implemented method of claim 2, wherein detecting the face of the subject comprises determining a bounding box for the face using a single shot multibox detector.

4. The computer-implemented method of any of claims 1 to 3, wherein detecting the body part in the image comprises:
generating a body skeleton (340) for the subject, the body skeleton comprising a plurality of landmarks and connecting lines between the landmarks, based on the image data,
wherein the connecting lines between the landmarks represent a first size of a corresponding body part; and
detecting the body part (345) based on the generated body skeleton; and
wherein determining the first size of the detected body part (350) is based on the length of a connecting line corresponding to the body part

5. The computer-implemented method of claim 4, wherein detecting the body part in the image comprises using a mask RCNN model to generate the body skeleton.

6. The computer-implemented method of any preceding claim, wherein determining the first size of the detected iris comprises determining a horizontal width of the detected iris.

7. The computer-implemented method of any preceding claim, wherein the method further comprises:
detecting an interpupillary distance (470) of the subject in the image;
determining a first size of the detected interpupillary distance (480) as represented in the image; and
wherein estimating the absolute size of the detected body part (460) is further based on the determined first size of the detected interpupillary distance and an absolute size of the interpupillary distance.

8. The computer-implemented method of claim 7, wherein the method further comprises receiving the absolute size of the interpupillary distance from a database based on the subject's age and sex.

9. The computer-implemented method of claim 7, wherein the method further comprises estimating the absolute size of the interpupillary distance (490) based on the first size of the detected interpupillary distance, the first size of the detected iris and the predetermined absolute iris size.

10. The computer-implemented method of any preceding claim, wherein the method further comprises:
determining an operating parameter (170) of a personal care device based on the estimated absolute size of the body part.

11. The computer-implemented method of claim 10, wherein the personal care device comprises an Intense Pulsed Light, IPL, device, and wherein the operating parameter comprises a number of flashes to be used on the body part.

12. The computer-implemented method of any preceding claim, wherein the image of the subject is a two-dimensional image.

13. A computer program product comprising computer program code means which, when executed on a processor, cause the computer-implemented method according to any of claims 1 to 12 to be performed.

14. A system (500) for estimating an absolute size of a body part, the system comprising:
a processor (520) configured to execute the computer program product according to claim 13.

15. A light-based skin treatment device (700) adapted to perform skin treatment, comprising:
a light source (800) adapted to generate treatment light (806) to perform skin treatment;
a controller (802); and
a data input (804) adapted to receive input data (808) from the system (500) according to claim 14,
wherein the input data (808) is representative of the estimated absolute size of the detected body part;
wherein the controller (802) is adapted to control the light-based skin treatment device (700) to perform skin treatment based on the estimated absolute size of the detected body part.
